# EUROPEAN PATENT APPLICATION

(11) **EP 2 014 219 A2**
(43) Date of publication of application: **14.01.2009**
(21) Application number: 08008777.8
(22) Date of filing: 09.05.2008
(51) Int. Cl.: A61B 1/04, A61B 19/00, G06T 7/00, G06F 19/00

(54) **Endoscopic image processing apparatus**

(30) Priority: 06.06.2007 JP 2007150921
(71) Applicant: Olympus Medical Systems Corp., Tokyo 151-0072 (JP)
(72) Inventor: Nishimura, Hirokazu, Tokyo 151-0072 (JP)
(74) Representative: von Hellfeld, Axel

(57) **Abstract**

An endoscopic image processing apparatus according to the present invention includes: an image acquiring unit which acquires images according to subject images picked up over time by an endoscope inserted into an object to be examined; a lesion detecting unit which detects a lesion in an image each time the image is acquired; a display unit which displays the images; and an image display control unit which controls display conditions of a plurality of images including at least a lesion image in which a lesion has been detected by the lesion detecting unit out of the images acquired by the image acquiring unit.

## Description

### BACKGROUND OF THE INVENTION

### 1. Field of the Invention

The present invention relates to an endoscopic image processing apparatus, and more particularly to an endoscopic image processing apparatus which individually controls display conditions of images according to subject images picked up over time by an endoscope inserted into an object to be examined.

### 2. Description of the Related Art

Conventionally, endoscope systems which include an endoscope are widely used in the industrial, medical, and other fields. In the medical field, in particular, endoscope systems are used mainly for applications such as observation of various organs in a living body. The endoscope systems used for the applications described above include, for example, an electronic endoscope system proposed in Japanese Patent Application Laid-Open No. 2006-223850.

The electronic endoscope system described in Japanese Patent Application Laid-Open No. 2006-223850 includes an image pickup unit which picks up images in a body of a subject using an image pickup device disposed in a distal end portion of an endoscope; a position detecting unit which acquires positional information about the distal end portion of an endoscope; a recording unit which records the images picked up by the image pickup unit as still images associated with the positional information acquired by the position detecting unit, using predetermined timing; and a display unit which displays the still images recorded in the recording unit and the positional information associated with the still images as well as displays the images being picked up by the image pickup unit as moving images together with the positional information being acquired by the position detecting unit. Being configured as described above, the electronic endoscope system described in Japanese Patent Application Laid-Open No. 2006-223850 makes it possible to identify locations of the images picked up by the image pickup unit.

On the other hand, regarding observation of the large intestine, in particular, among various types of observation made by means of an endoscope, it is conceivable that a technique in which, for example, a surgeon observes lesion by withdrawing an endoscope inserted by a nurse or other assistant into the ileocecum, the innermost part of the large intestine, in advance will likely be realized in the future. Even today, skilled surgeons often use a technique in which the surgeons make detailed observations, give treatments, and so on by withdrawing an endoscope inserted into the ileocecum by the surgeons themselves.

When using the observation technique described above, the surgeon must pay maximum attention during withdrawal of the endoscope, for example, in order not to omit a lesion noticed during insertion of the endoscope. Thus, with the observation techniques described above, a task is to realize a more efficient way of preventing a lesion noticed during insertion of the endoscope from being omitted while reducing burdens on the surgeon.

On the other hand, the electronic endoscope system described in Japanese Patent Application Laid-Open No. 2006-223850 is configured to associate each image with the positional information about the distal end portion of the endoscope at the time when the image is picked up, but is not configured, for example, to allow information about the lesion noticed during insertion of the endoscope to be provided during withdrawal of the endoscope in order to prevent omission of the lesion, which can occur when the observation technique described above is used. In other words, the electronic endoscope system described in Japanese Patent Application Laid-Open No. 2006-223850 is not configured to solve the problem with the observation technique described above.

The present invention has been made in view of the above circumstances and has an object to provide an endoscopic image processing apparatus which allows lesion information acquired during insertion of the endoscope to be provided during withdrawal of the endoscope and thereby improves efficiency of observation by means of an endoscope.

### SUMMARY OF THE INVENTION

An endoscopic image processing apparatus according to the present invention includes: an image acquiring unit which acquires images according to subject images picked up over time by an endoscope inserted into an object to be examined; a lesion detecting unit which detects a lesion in an image each time the image is acquired; a display unit which displays the images; and an image display control unit which controls display conditions of a plurality of images including at least a lesion image in which a lesion has been detected by the lesion detecting unit out of the images acquired by the image acquiring unit.

Preferably, in the endoscopic image processing apparatus according to the present invention, the image display control unit makes the display unit display images during a predetermined period around the time when the image acquiring unit acquires the lesion image.

Preferably, in the endoscopic image processing apparatus according to the present invention, out of a series of images acquired by the image acquiring unit from a start to a completion of insertion of the endoscope and arranged in chronological, the image display control unit causes the lesion image to be displayed as a still image and the other images to be played back as moving images.

Preferably, in the endoscopic image processing apparatus according to the present invention, the image display control unit causes the images other than the lesion image to be played back as moving images at a higher speed than image pickup speed of the endoscope.

Preferably, in the endoscopic image processing apparatus according to the present invention, out of a series of images acquired by the image acquiring unit from a start to a completion of insertion of the endoscope and arranged in reverse chronological order, the image display control unit causes the lesion image to be displayed as a still image and the other images to be played back in reverse as moving images.

Preferably, in the endoscopic image processing apparatus according to the present invention, the image display control unit causes the images other than the lesion image to be played back in reverse as moving images at a higher speed than image pickup speed of the endoscope.

Preferably, in the endoscopic image processing apparatus according to the present invention, out of a series of images acquired by the image acquiring unit from a start to a completion of insertion of the endoscope, the image display control unit causes the lesion image and a predetermined number of images temporally before and/or after the lesion image to be played back as moving images.

Preferably, in the endoscopic image processing apparatus according to the present invention, out of a series of images acquired by the image acquiring unit from a start to a completion of insertion of the endoscope, the image display control unit causes the lesion image and a predetermined number of images temporally before and/or after the lesion image to be played back in reverse as moving images.

Preferably, the endoscopic image processing apparatus according to the present invention further including an insertion status information acquiring unit which acquires insertion status data representing insertion status of the endoscope inserted into the object to be examined from an endoscope insertion status detecting apparatus and outputs information about the insertion status of the endoscope to the display unit together with the lesion image, the information about the insertion status of the endoscope corresponding to the insertion status data at the time when the image acquiring unit acquires the lesion image.

Preferably, in the endoscopic image processing apparatus according to the present invention, the information about the insertion status of the endoscope includes at least one of insertion length of the endoscope, elapsed time after insertion of the endoscope, and insertion shape of the endoscope.

### BRIEF DESCRIPTION OF THE DRAWINGS

Fig. 1 is a diagram showing an exemplary configuration of a principal part of a body imaging system in which an image processing apparatus according to the present embodiment is used;
Fig. 2 is a diagram showing coordinates of source coils detected by an endoscope insertion status detecting apparatus in Fig. 1, the source coils being installed in an insertion portion of an endoscope in Fig. 1;
Fig. 3 is a flowchart showing a part of a process performed by the image processing apparatus shown in Fig. 1 to detect an elevated lesion;
Fig. 4 is a flowchart showing the process performed subsequently to that in Fig. 3 by the image processing apparatus shown in Fig. 1 to detect the elevated lesion;
Fig. 5 is a diagram showing an example of a three-dimensional model estimated by the image processing apparatus shown in Fig. 1;
Fig. 6 is a diagram showing an example of a region containing a voxel group used to detect the elevated lesion in the three-dimensional model shown in Fig. 5;
Fig. 7 is a diagram showing an example of images and the like presented on a display panel of the image processing apparatus shown in Fig. 1 when a lesion has been detected;
Fig. 8 is a diagram showing an example of a method for displaying an endoscopic image on the display panel of the image processing apparatus shown in Fig. 1; and
Fig. 9 is a diagram showing another example of a method for displaying an endoscopic image on the display panel of the image processing apparatus shown in Fig. 1.

### DETAILED DESCRIPTION OF THE PREFERRED EMBODIMENTS

Preferred embodiments of the present invention will be described below with reference to the drawings.

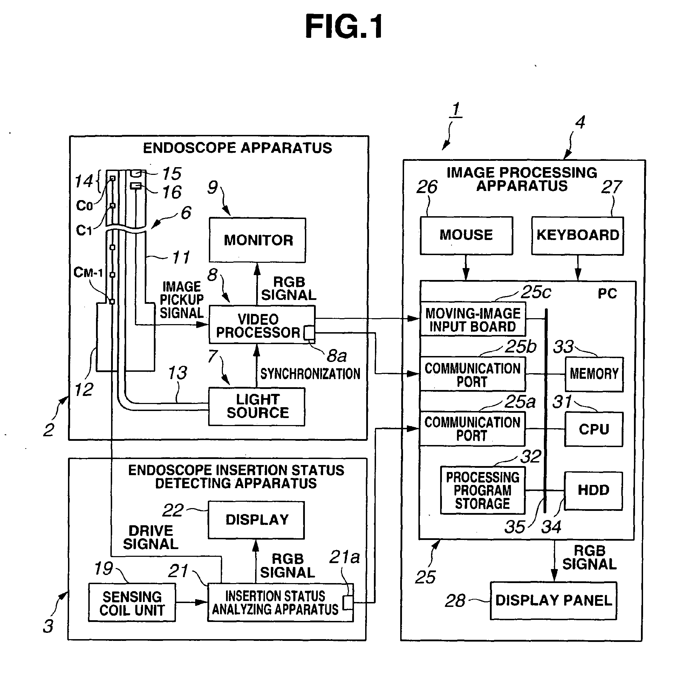

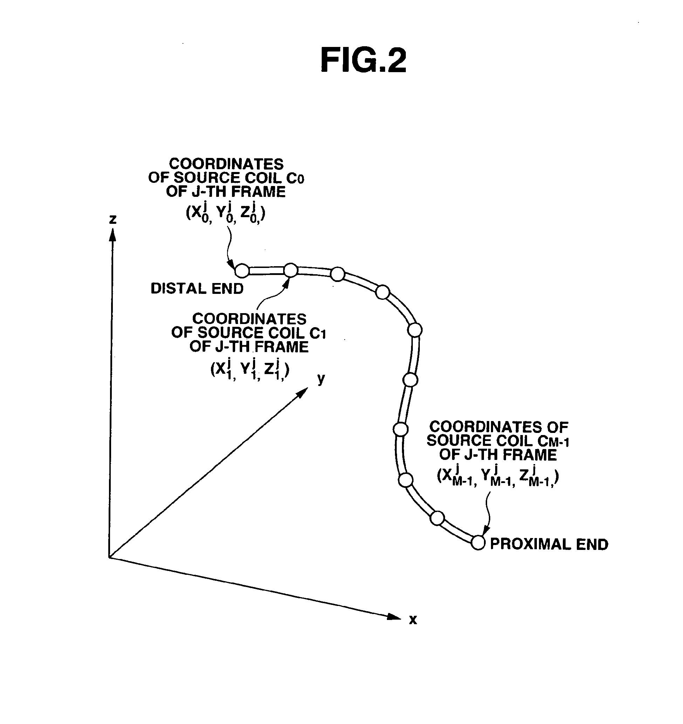

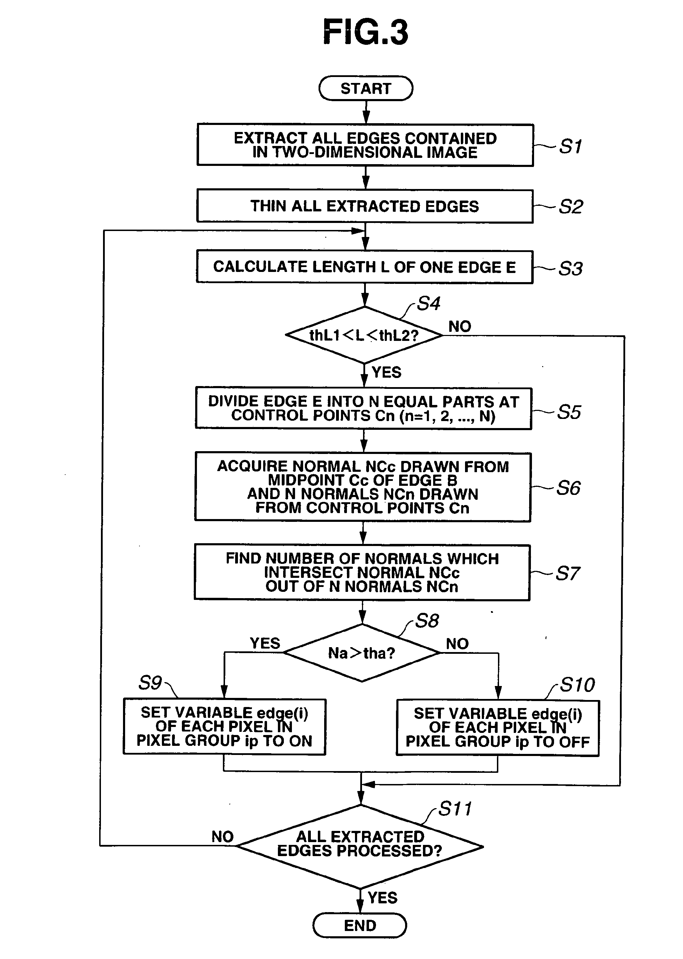

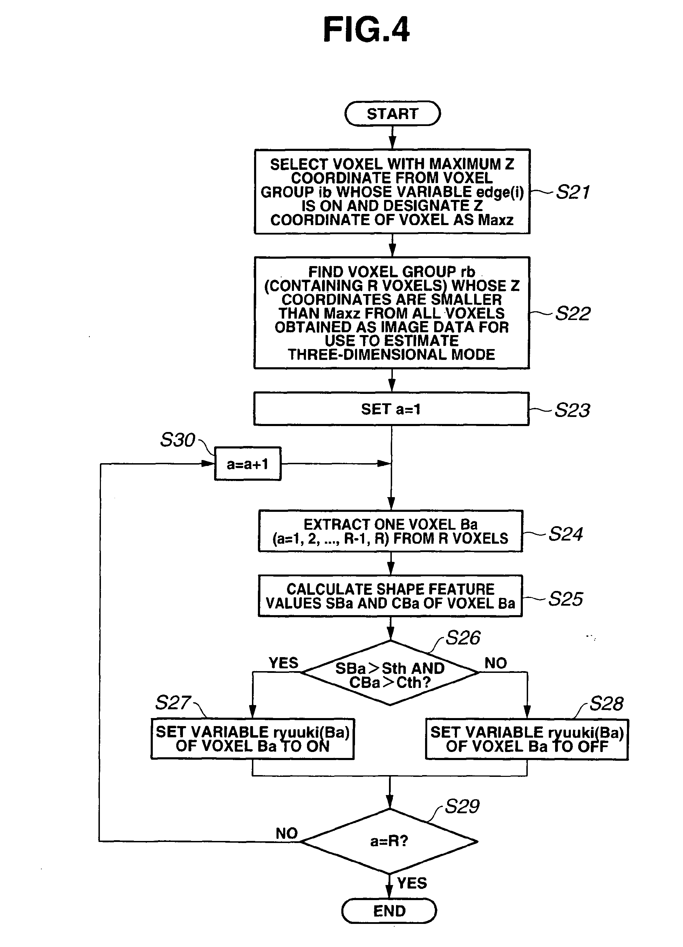

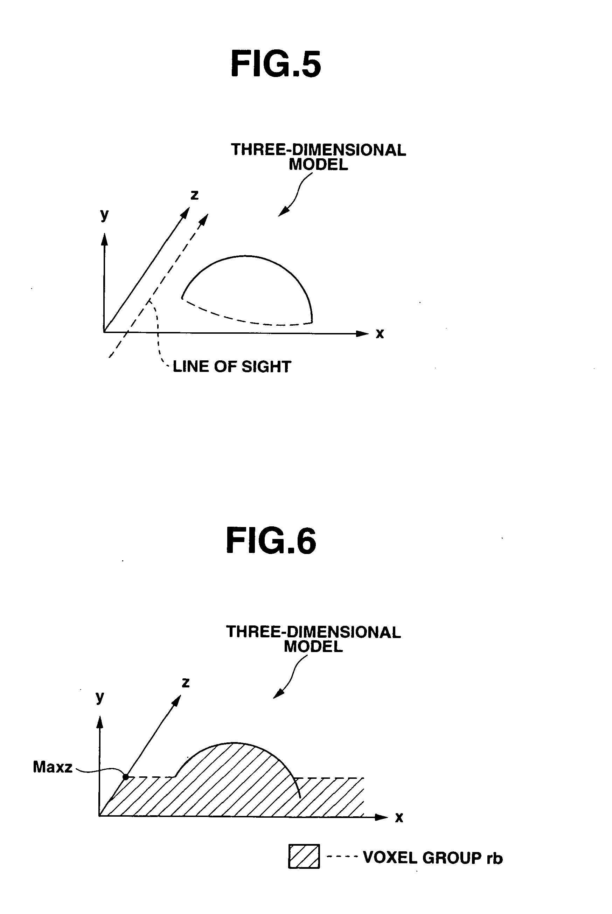

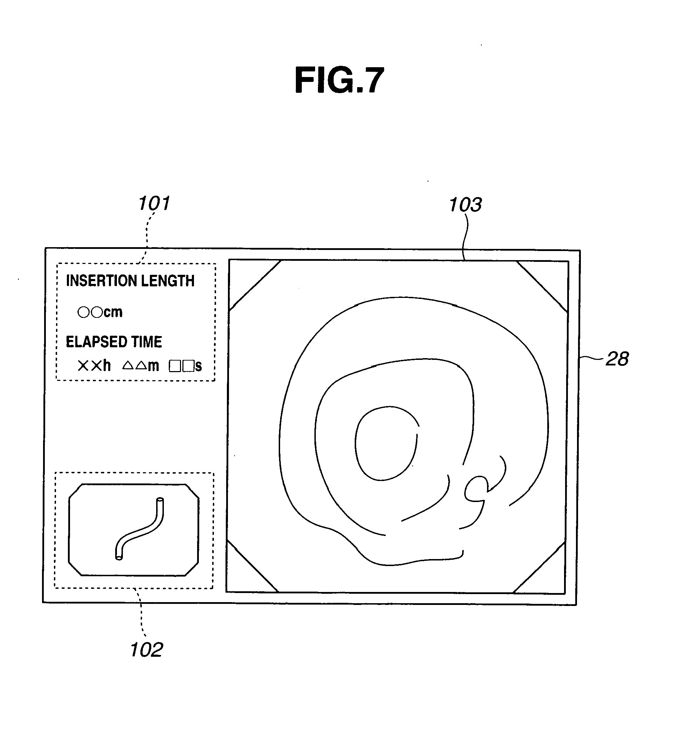

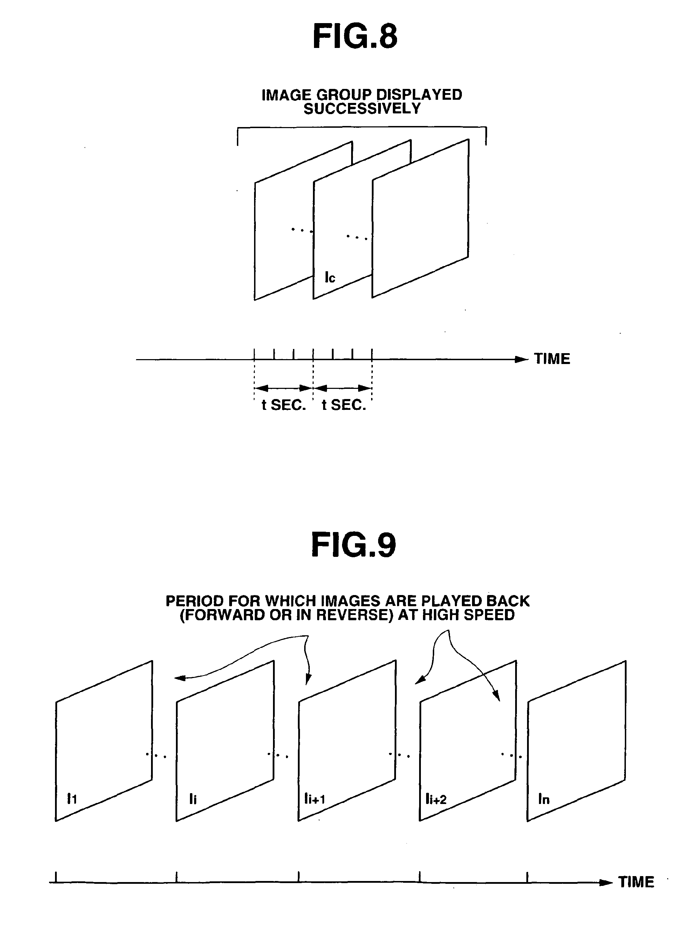

Figs. 1 to 9 relate to an embodiment of the present invention. Fig. 1 is a diagram showing an exemplary configuration of a principal part of a body imaging system in which an image processing apparatus according to the present embodiment is used. Fig. 2 is a diagram showing coordinates of source coils detected by an endoscope insertion status detecting apparatus in Fig. 1, the source coils being installed in an insertion portion of an endoscope in Fig. 1. Fig. 3 is a flowchart showing a part of a process performed by the image processing apparatus shown in Fig. 1 to detect an elevated lesion. Fig. 4 is a flowchart showing the process performed subsequently to that in Fig. 3 by the image processing apparatus shown in Fig. 1 to detect the elevated lesion. Fig. 5 is a diagram showing an example of a three-dimensional model estimated by the image processing apparatus shown in Fig. 1. Fig. 6 is a diagram showing an example of a region containing a voxel group used to detect the elevated lesion in the three-dimensional model shown in Fig. 5. Fig. 7 is a diagram showing an example of images and the like presented on a display panel of the image processing apparatus shown in Fig. 1 when a lesion has been detected. Fig. 8 is a diagram showing an example of a method for displaying an endoscopic image on the display panel of the image processing apparatus shown in Fig. 1. Fig. 9 is a diagram showing another example of a method for displaying an endoscopic image on the display panel of the image processing apparatus shown in Fig. 1.

As shown in Fig. 1, a body imaging system 1 includes an endoscope apparatus 2 which allows a surgeon to observe internal body parts of a subject through an endoscope 6, an endoscope insertion status detecting apparatus 3 which detects insertion status of the endoscope 6 inserted into the internal body parts of the subject and outputs the insertion status as insertion status data, and an image processing apparatus 4 which performed various processes according to the insertion status data outputted from the endoscope insertion status detecting apparatus 3.

The endoscope apparatus 2 includes the endoscope 6 which, being able to be inserted into the large intestine of a subject, picks up images of an imaging subject in the subject and outputs a resulting image pickup signal, a light source 7 which supplies the endoscope 6 with illuminating light for use to illuminate the imaging subject, a video processor 8 which processes the image pickup signal outputted from the endoscope 6 and outputs a resulting video signal, and a monitor 9 which displays subject images picked up by the endoscope 6 as endoscopic images based on the video signal outputted from the video processor 8.

The endoscope 6 includes an insertion portion 11 and an operation portion 12 installed at a rear end of the insertion portion 11. A light guide 13 is passed through the insertion portion 11 with one end being located in a distal end portion 14 of the insertion portion 11 and the other end being connectable to the light source 7. Consequently, the illuminating light supplied by the light source 7 is emitted via the light guide 13 from an illuminating window (not shown) provided in a distal end portion 14 of the insertion portion 11.

Incidentally, a bending portion (not shown) configured to be bendable is installed on the rear end of the distal end portion 14 of the insertion portion 11. The bending portion (not shown) can be bent using a bending operation knob or the like (not shown) installed on the operation portion 12.

Next to the illuminating window (not shown) in the distal end portion 14, an objective lens 15 is mounted in an observation window (not shown). Also, an image pickup surface of an image pickup device 16 which includes a charge-coupled device (abbreviated to CCD) is located at an image-forming position of the objective lens 15.

The image pickup device 16, which is connected to the video processor 8 via a signal line, picks up images of the subject formed by the objective lens 15 and outputs a resulting image pickup signal to the video processor 8.

The video processor 8 performs signal processing to generate a video signal based on the image pickup signal outputted from the image pickup device 16. Then the video processor 8 outputs the generated video signal to the monitor 9, for example, in the form of an RGB signal. Consequently, the subject images picked up by the image pickup device 16 are displayed on a display screen of the monitor 9 as endoscopic images.

When supplying a surface-sequential illuminating light consisting, for example, of R (red), G (green), and B (blue), the light source 7 outputs, to the video processor 8, a synchronizing signal synchronized with periods for which individual colors are supplied. In so doing, the video processor 8 performs the signal processing in sync with the synchronizing signal outputted from the light source 7.

In addition to the bending operation knob (not shown), the operation portion 12 of the endoscope 6 contains a switch used to give a release command and the like.

Also, multiple source coils C₀, C₁, ..., C_{M-1} (C₀ to C_{M-1}) are located at predetermined intervals in a longitudinal direction in the insertion portion 11 of the endoscope 6. Each of the source coils C₀ to C_{M-1} generates a magnetic field around the given source coil according to a drive signal outputted by the endoscope insertion status detecting apparatus 3.

The magnetic fields emitted from the source coils C₀ to C_{M-1} are detected by a sensing coil unit 19 of the endoscope insertion status detecting apparatus 3, the sensing coil unit 19 containing multiple sensing coils.

The endoscope insertion status detecting apparatus 3 includes the sensing coil unit 19 which detects the magnetic fields emitted from the source coils C₀ to C_{M-1} of the endoscope 6, an insertion status analyzing apparatus 21 which can estimate a shape of the insertion portion 11 and otherwise analyze insertion status of the insertion portion 11 based on a detection signal about the magnetic fields detected by the sensing coil unit 19, and a display 22 which displays the shape of the insertion portion 11 estimated by the insertion status analyzing apparatus 21.

The sensing coil unit 19, which is located, for example, around a bed on which a patient lies, detects the magnetic fields of the source coils C₀ to C_{M-1} and outputs the detection signal about the detected magnetic fields to the insertion status analyzing apparatus 21.

Based on the detection signal, the insertion status analyzing apparatus 21 calculates position coordinate data of each of the source coils C₀ to C_{M-1} and estimates an insertion shape of the insertion portion 1 I based on the calculated position coordinate data. Also, the insertion status analyzing apparatus 21 generates a video signal of the estimated insertion shape of the insertion portion 11 and outputs the generated video signal to the display 22, for example, in the form of an RGB signal. Consequently the insertion shape of the insertion portion 11 is presented on the display 22. Furthermore, during observation via the endoscope 6, the insertion status analyzing apparatus 21 continuously generates insertion status information about the shape of the insertion portion 11, insertion length of the insertion portion 11, elapsed time after insertion of the insertion portion 11, shape display properties, and the like and outputs the insertion status information to the image processing apparatus 4 via a communications port 21 a.

Also, when an image of the insertion shape is presented on the display 22 after a shape detection process of the insertion status analyzing apparatus 21, the endoscope insertion status detecting apparatus 3 according to the present embodiment allows the surgeon to change the shape display properties, such as a rotation angle and zoom ratio, of the image of the insertion shape by entering commands and the like on an operation panel (not shown).

Incidentally, the video processor 8 has an operation panel (not shown) for use to enter inspection information including patient's name, date of birth, sex, age, patient code, and inspection date/time. The inspection information entered through the operation panel is outputted to the monitor 9, being superimposed over the video signal generated by the video processor 8, and is transmitted to the image processing apparatus 4 via a communications port 8a.

The image processing apparatus 4 serving as the endoscopic image processing apparatus includes a personal computer 25 (hereinafter simply referred to as a 'PC') which performs various processes based on the insertion status data outputted from the endoscope insertion status detecting apparatus 3 and the inspection information outputted from the video processor 8; a mouse 26 and a keyboard 27 used to enter various commands and inputs in the PC 25; and a display panel 28 which displays images, information, and the like generated as a result of the various processes of the PC 25.

The PC 25 includes a communications port 25a used to capture the insertion status data outputted from the communications port 21 a of the insertion status analyzing apparatus 21 of the endoscope insertion status detecting apparatus 3, a communications port 25b used to capture the inspection information outputted from the communications port 8a of the video processor 8, a moving-image input board 25c which converts a video signal of moving images generated by the video processor 8 into compressed image data in predetermined format, a CPU 31 which performs various types of signal processing, a processing program storage 32 which stores processing programs used by the CPU 31 for the various types of signal processing, a memory 33 which stores data processed by the CPU 31, and a hard disk (hereinafter simply referred to as an 'HDD') 34 which stores image data and the like processed by the CPU 31. Respective various components of the PC 25 are interconnected via a busline 35 with one another.

The video signal of moving images generated by the video processor 8 is inputted in the moving-image input board 25c of the image processing apparatus 4, for example, in the form of a Y/C signal with a predetermined frame rate (30 frames/second). The moving-image input board 25c converts the video signal of the moving images into compressed image data in a predetermined compression format such as MJPEG format and outputs the compressed image data to the HDD 34 and the like.

The insertion status data captured through communications port 25a and the inspection information captured through the communications port 25b are outputted, for example, to the memory 33 and thereby stored in the PC 25.

The display panel 28, which has functions similar to functions of a touch panel, is able to display images and information generated through various processes of the PC 25 and output entries related to the displayed images to the PC 25 in the form of a signal.

Now, processes performed by the endoscope insertion status detecting apparatus 3 to generate the insertion status data will be described.

Each time an image pickup signal of one frame is outputted from the image pickup device 16 of the endoscope 6, the insertion status analyzing apparatus 21 of the endoscope insertion status detecting apparatus 3 generates insertion status data including three-dimensional coordinates of M source coils Co to C_{M-1} incorporated in the insertion portion 11. Also, the insertion status analyzing apparatus 21 outputs the insertion status data to the image processing apparatus 4 and generates an image of an insertion shape of the insertion portion 11 and outputs the image of the insertion shape to the display 22.

Incidentally, the three-dimensional coordinates of the i-th (i = 0, 1,... M-1) source coil Cᵢ from distal end of the insertion portion 11 in the j-th frame (j = 0, 1, 2 ...) are expressed as X^{j}₁, Y^{j}₁, Z^{j}₁ as shown in Fig. 2.

The insertion status data detected by the endoscope insertion status detecting apparatus 3 including data on a coordinate system of the source coils C₀ to C_{M-1} is configured as frame data of individual frames (0-th frame data, first-frame data,...) and transmitted to the image processing apparatus 4 in sequence. The frame data of each frame includes creation time, display properties, associated information and (three-dimensional) source coil coordinates, and the like.

Coil coordinate data represents the three-dimensional coordinates of the source coils C₀ to C_{M-1} arranged in order from distal end to proximal end (on the side of the operation portion 12) of the insertion portion 11. Three-dimensional coordinates of source coils outside a detection range of the endoscope insertion status detecting apparatus 3 are represented, for example, by predetermined coordinate values (such as 0, 0, 0) so that it can be seen that the source coils are located outside the detection range.

Next, operation of the body imaging system 1 according to the present embodiment will be described.

When the insertion portion 11 of the endoscope 6 is inserted from the anus into the body cavity of the subject by an assistant such as a nurse or engineer, an image of an imaging subject in the body cavity is picked up by the image pickup device 16 attached to the distal end portion 14 of the insertion portion 11. The subject images are picked up over time by the image pickup device 16 and outputted as an image pickup signal. Subsequently, the image pickup signal is converted into a video signal through signal processing performed by the video processor 8 and outputted to the monitor 9. Consequently, the subject image picked up by the image pickup device 16 is displayed on the monitor 9 as an endoscopic image.

The endoscope insertion status detecting apparatus 3 detects the respective magnetic fields of the source coils C₀ to C_{M-1} using the sensing coil unit 19 and estimates the insertion shape of the insertion portion 11 using the insertion status analyzing apparatus 21 based on the detection signal outputted according to the magnetic fields. Consequently, the insertion shape of the insertion portion 11 estimated by the insertion status analyzing apparatus 21 is presented on the display 22.

The video signal generated by the video processor 8 is outputted to the CPU 31 via the communications ports 8a and 25b.

The CPU 31, which functions as an image acquiring unit and lesion detecting unit, acquires an image according to a subject image picked up by the endoscope 6 based on the inputted video signal and a processing program written in the processing program storage 32. Each time such an image is acquired, the CPU 31 performs a process intended to detect a lesion in the image.

Now, a series of processes performed by the CPU 31 to detect an elevated lesion in the subject image picked up by the endoscope 6 will be described. It is assumed that the lesion detection processes described below are performed on an image of each frame in the video signal outputted from the video processor 8.

First, based on the inputted video signal, the CPU 31 extracts all edges contained in the subject image picked up by the endoscope 6 and makes thinner outline thereof, and then calculates length L of one edge E out of all the thinned edges (Steps S1, S2, and S3 in Fig. 3). Furthermore, the CPU 31 determines whether or not the length L of the edge E is longer than a threshold thL 1 and shorter than a threshold thL2 (Step S4 in Fig. 3).

If it is found that the length L of the edge E is equal to or shorter than the threshold thL1 or that the length L of the edge E is equal to or longer than the threshold thL2, the CPU 31 determines that the edge E is not traceable to a lesion and performs a process of Step S3 described later. On the other hand, if it is found that the length L of the edge E is longer than the threshold thL1 and shorter than the threshold thL2, the CPU 31 divides the edge E into N equal parts at control points Cn (n = 1, 2, ..., N) (Step S5 in Fig. 3).

Furthermore, the CPU 31 acquires a normal NCc drawn from the midpoint Cc of the edge E and N normals NCn drawn from the control points Cn (Step S6 in Fig. 3). Subsequently, out of the N normals NCn, the CPU 31 finds the number of normals which intersects the normal NCc (Step S7 in Fig. 3).

Also, the CPU 31 determines whether or not the number of normals which intersects the normal NCc out of the N normals NCn is larger than a threshold tha (Step S8 in Fig. 3). If it is found that the number of normals which intersects the normal NCc is larger than that of the threshold tha, the CPU 31 determines that a pixel group ip contained in the edge E is included in an edge of a candidate for a lesion and sets a value of a variable edge(i) of each pixel in the pixel group ip to ON (Step S9 in Fig. 3). On the other hand, if it is found that the number of normals which intersect the normal NCc is equal to or smaller than the threshold tha, the CPU 31 determines that the pixel group ip contained in the edge E is not included in an edge traceable to a lesion, and sets the value of the variable edge(i) of each pixel in the pixel group ip to OFF (Step S10 in Fig. 3).

Subsequently, the CPU 31 determines whether or not all the extracted edges have been processed (Step S 11 in Fig. 3). If it is found that all the extracted edges have not been processed, the CPU 31 performs the processes of Steps S3 to S 10 described above on another edge. On the other hand, if it is found that all the extracted edges have been processed, the CPU 31 finishes the series of processes for detecting edges in a two-dimensional image.

The CPU 31 temporarily stores the values of the variable edge(i) of the pixel group ip in the memory 33 as a result of the series of processes performed on all the extracted edges.

The CPU 31 acquires image data needed to estimate a three-dimensional model of the subject image of the imaging subject picked up by the endoscope 6 by performing processes such as geometric transformations based on luminance information and the like in the video signal outputted from the video processor 8. In other words, the CPU 31 generates a voxel corresponding to each pixel in the two-dimensional image through processes such as geometric transformations and acquires the voxel as image data for use to estimate the three-dimensional model. That is, the pixel group ip is converted into a voxel group ib through the processes described above.

Through the processes described above, the CPU 31 acquires data of a boundary plane as image data needed to estimate the three-dimensional model of the subject image picked up by the endoscope 6, where the boundary plane is a plane which includes the voxel group ib whose variable edge(i) is ON. Consequently, the subject image picked up by the endoscope 6 is estimated as a three-dimensional model of a shape such as shown in Fig. 5 if a z-axis direction corresponds to a line of sight during observation through the endoscope 6.

Subsequently, based on the data of the boundary plane, the CPU 31 selects a voxel with a maximum z coordinate as a predetermined innermost voxel along the line of sight of the endoscope 6 from the voxel group ib whose variable edge(i) is ON and designates the z coordinate of the voxel as Maxz (Step S21 in Fig. 4).

Next, as voxels located on the near side of the innermost voxel along the line of sight of the endoscope 6, the CPU 31 finds a voxel group rb whose z coordinates are smaller than Maxz from all the voxels obtained as image data for use to estimate the three-dimensional model of the subject image picked up by the endoscope 6 (Step S22 in Fig. 4). Incidentally, the voxel group rb is made up of R voxels existing, for example, in a region shown in Fig. 6.

Furthermore, the CPU 31 sets a variable a to 1, extracts one voxel Ba (a = 1, 2, ..., R-1, R) from the R voxels in the voxel group rb, and calculates a ShapeIndex value SBa and Curvedness value CBa as shape feature values of the voxel Ba (Steps S23, S24, and S25 in Fig. 4).

Incidentally, the ShapeIndex value SBa and Curvedness value CBa described above can be calculated using a method similar to a method described, for example, in US Patent Application Publication No. 20030223627. Thus, description of the method for calculating the ShapeIndex value SBa and Curvedness value CBa in one voxel Ba will be omitted according to the present embodiment.

Also, the CPU 31 compares the ShapeIndex value SBa with a predetermined threshold Sth (e.g., 0.9) of the ShapeIndex value and compares the Curvedness value CBa with a predetermined threshold Cth (e.g., 0.2) of the Curvedness value (Step S26 in Fig. 4). In other words, the CPU 31 extracts a voxel group whose three-dimensional model is estimated to have a convex shape to detect whether or not the subject image picked up by the endoscope 6 shows an elevated lesion.

If it is found that the ShapeIndex value SBa is larger than the threshold Sth and that the Curvedness value CBa is larger than the threshold Cth, the CPU 31 determines that the voxel Ba is a part of an elevated lesion and sets a value of a variable ryuuki(Ba) of the voxel Ba to ON (Step S27 in Fig. 4).

On the other hand, if it is detected that the ShapeIndex value SBa is equal to or smaller than the threshold Sth or that the Curvedness value CBa is equal to or smaller than the threshold Cth, the CPU 31 determines the voxel Ba is not a part of an elevated lesion and sets the value of the variable ryuuki(Ba) of the voxel Ba to OFF (Step S28 in Fig. 4).

Subsequently, the CPU 31 determines whether or not all the R voxels have been processed, i.e., whether or not the variable a = R (Step S29 in Fig. 4).

If it is found that a is not equal to R, the CPU 31 adds 1 to the variable i (Step S30 in Fig. 4), and then repeats the processes of Steps S24 to S29.

If it is found that a = R (Step S29 in Fig. 4), the CPU 31 finishes the series of processes for detecting an elevation in the three-dimensional model of the subject image picked up by the endoscope 6.

The CPU 31 temporarily stores the values of ryuuki(Ba) in the memory 33 as a result of the series of processes performed on all the R voxels.

Next, in the two-dimensional image, the CPU 31 detects a pixel located at a position corresponding to a position of each voxel whose ryuuki(Ba) value is ON.

By performing the above processes with respect to an image of each frame in the video signal outputted from the video processor 8, the CPU 31 detects any elevated lesion, such as a polyp, contained in the subject image picked up by the endoscope 6.

Furthermore, based on the video signal outputted from the video processor 8, lesion detection results produced in the series of processes, and insertion status data inputted via the communications ports 21a and 25a, the CPU 31, which functions as an image display control unit and insertion status information acquiring unit, acquires various information and stores the various information in the HDD 34 by correlating the various information as well as displays the various information on the display panel 28 by reading the information out of the HDD 34 with predetermined timing, where the various information includes, for example, the image of a scene in which a lesion was detected, insertion shape and insertion length of the insertion portion 11 at the time when the lesion was detected, and elapsed time from the insertion of the insertion portion 11 to the acquisition of the image. As a result of the above-described operations performed under the control of the CPU 31, the display panel 28 simultaneously displays information such as shown in Fig. 7: insertion status information 101 which includes at least the insertion length and elapsed time, an inserted-shape image 102 which shows the insertion shape of the insertion portion 11 at the time when the lesion was detected, and an endoscopic image 103 of the scene in which the lesion was detected. Incidentally, the display panel 28 may display at least one of them, but not necessarily display all of the various information contained in the insertion status information 101 and the inserted-shape image 102 (as shown in Fig. 7).

Regarding the predetermined timing, the various information may be displayed immediately after a lesion is detected during insertion of the insertion portion 11 or when an insertion-complete button (not shown) of the endoscope 6 is pressed after the distal end portion 14 of the insertion portion 11 reaches the ileocecum.

Contents displayed on the display panel 28 are not limited to those shown in Fig. 7. For example, if multiple lesions are detected, thumbnail images of endoscopic images 103 may be listed first and an image selected from the thumbnail images may be displayed in a manner shown in Fig. 7. Incidentally, order of the listing may be based, for example, on at least one of detection time of the lesion, insertion length, and elapsed time.

The operation described above allows the surgeon to check for lesions and determine the number and approximate locations of the lesions before the assistant finishes inserting the insertion portion 11. Furthermore, the operation described above allows the surgeon to make observations with reference to the endoscopic images 103 displayed on the display panel 28 while the surgeon withdraws the insertion portion 11.

According to the present embodiment, the image processing apparatus 4 may be configured to mark images of detected lesions during insertion of the insertion portion 11 and alert the surgeon when the distal end portion 14 approaches a site which corresponds to each marked image during withdrawal of the insertion portion 11.

The endoscopic images 103 displayed on the display panel 28 are not limited to still images of scenes in which lesions have been detected. As shown in Fig. 8, moving images may be displayed successively under the control of the CPU 31 for t seconds before and after acquisition of a still image I_{c} of each scene in which a lesion has been detected.

Specifically, for example, out ofN images I₁ to In acquired during insertion of the insertion portion 11, the CPU 31 serving as an image display control unit may cause a predetermined number of images acquired in t seconds before and/or after the acquisition of the still image I_{c} to be displayed (played back forward or in reverse) successively together with the still image I_{c}.

The endoscopic images 103 displayed on the display panel 28 are not limited to still images of scenes in which lesions have been detected. For example, the N images I₁ to In acquired during insertion of the insertion portion 11 may be played back as moving images in digest form under the control of the CPU 31.

The digest playback is achieved, for example, as follows: out of the N images I₁ to In arranged in chronological order as moving images, the images of the scenes in which lesions have been detected are displayed as paused images (still images) on the display panel 28 (in a display section for endoscopic images 103) and the other images are played back at high speed on the display panel 28 (in the display section for endoscopic images 103). For example, as shown in Fig. 9, if images Iᵢ, Iᵢ₊₁, and Iᵢ₊₂ are acquired out of the N images I₁ to In as the images of the scenes in which lesions have been detected, under the control of the CPU 31, the images Iᵢ, Iᵢ₊₁, and Iᵢ₊₂ are displayed as paused images (still images) on the display panel 28 (in the display section for endoscopic images 103) out of the series of images from the image I₁ at the start of insertion of the insertion portion 11 to the image In at the completion of the insertion and the other images are played back at high speed on the display panel 28 (in the display section for endoscopic images 103). Incidentally, speed of the high-speed playback is higher than, for example, image pickup speed of the image pickup device 16 of the endoscope 6.

Furthermore, the endoscopic images 103 displayed on the display panel 28 are not limited to the still images of scenes in which lesions have been detected, and the N images I₁ to In acquired during insertion of the insertion portion 11 may be played back in reverse as moving images in digest form under the control of the CPU 31.

The digest playback in reverse is achieved, for example, as follows: out of the N images In to I₁ arranged in reverse chronological order as moving images, the images of the scenes in which lesions have been detected are displayed as paused images (still images) on the display panel 28 (in the display section for endoscopic images 103) and the other images are played back at high speed on the display panel 28 (in the display section for endoscopic images 103). For example, as shown in Fig. 9, if images Iᵢ, Iᵢ₊₁, and Iᵢ₊₂ are acquired out of the N images I₁ to In as the images of the scenes in which lesions have been detected, under the control of the CPU 31, the images Iᵢ₊₂, Iᵢ₊₁, and Iᵢ are displayed as paused images (still images) on the display panel 28 (in the display section for endoscopic images 103) out of the series of images from the image In at the completion of the insertion of the insertion portion 11 to the image I₁ at the start of insertion and the other images are played back at high speed on the display panel 28 (in the display section for endoscopic images 103). Speed of the high-speed playback in reverse is higher than, for example, image pickup speed of the image pickup device 16 of the endoscope 6.

As described above, the image processing apparatus 4 according to the present embodiment (the body imaging system 1 equipped with the image processing apparatus 4) is configured to allow the images of, and information about, the scenes in which lesions have been detected to be displayed on the display panel 28 during (or before) the insertion portion 11 is withdrawn. Consequently, the image processing apparatus 4 according to the present embodiment (the body imaging system 1 equipped with the image processing apparatus 4) can improve the efficiency of observation by means of an endoscope. Advantages described above are especially pronounced in the case of an observation technique in which an endoscope is inserted and withdrawn by different persons.

Also, the image processing apparatus 4 according to the present embodiment (the body imaging system 1 equipped with the image processing apparatus 4) offers the advantages described above, for example, when the surgeon makes observations by moving the endoscope back and forth near a desired site.

The image processing apparatus 4 according to the present embodiment (the body imaging system 1 equipped with the image processing apparatus 4) is configured to be able to detect elevated lesions such as polyps through image processing. And the image processing apparatus 4 according to the present embodiment (the body imaging system 1 equipped with the image processing apparatus 4) may also be configured to allow an operator of the endoscope 6 to press lesion-detected button or the like (not shown) upon detection of a lesion, thereby making the CPU 31 recognize the existence of the lesion.

The present invention is not limited to the embodiment described above, and various modifications and applications are possible without departing from the spirit of the present invention.

## Claims

1. An endoscopic image processing apparatus comprising:
an image acquiring unit which acquires images according to subject images picked up over time by an endoscope inserted into an object to be examined;
a lesion detecting unit which detects a lesion in an image each time the image is acquired;
a display unit which displays the images; and
an image display control unit which controls display conditions of a plurality of images including at least a lesion image in which a lesion has been detected by the lesion detecting unit out of the images acquired by the image acquiring unit.

2. The endoscopic image processing apparatus according to claim 1, wherein the image display control unit makes the display unit display images of a predetermined period around the time when the image acquiring unit acquires the lesion image.

3. The endoscopic image processing apparatus according to claim 1, wherein out of a series of images acquired by the image acquiring unit from a start to a completion of insertion of the endoscope and arranged in chronological order, the image display control unit causes the lesion image to be displayed as a still image and the other images to be played back as moving images.

4. The endoscopic image processing apparatus according to claim 3, wherein the image display control unit causes the images other than the lesion image to be played back as moving images at higher speed than image pickup speed of the endoscope.

5. The endoscopic image processing apparatus according to claim l, wherein, out of a series of images acquired by the image acquiring unit from a start to a completion of insertion of the endoscope and arranged in reverse chronological order, the image display control unit causes the lesion image to be displayed as a still image and the other images to be played back in reverse as moving images.

6. The endoscopic image processing apparatus according to claim 5, wherein the image display control unit causes the images other than the lesion image to be played back in reverse as moving images at a higher speed than image pickup speed of the endoscope.

7. The endoscopic image processing apparatus according to claim 1, wherein out of a series of images acquired by the image acquiring unit from a start to a completion of insertion of the endoscope, the image display control unit causes the lesion image and a predetermined number of images temporally before and/or after the lesion image to be played back as moving images.

8. The endoscopic image processing apparatus according to claim 1, wherein out of a series of images acquired by the image acquiring unit from a start to a completion of insertion of the endoscope, the image display control unit causes the lesion image and a predetermined number of images temporally before and/or after the lesion image to be played back in reverse as moving images.

9. The endoscopic image processing apparatus according to any one of claims 1 to 8, further comprising an insertion status information acquiring unit which acquires insertion status data representing insertion status of the endoscope inserted into the object to be examined from an endoscope insertion status detecting apparatus and outputs information about the insertion status of the endoscope to the display unit together with the lesion image, the information about the insertion status of the endoscope corresponding to the insertion status data at the time when the image acquiring unit acquires the lesion image.

10. The endoscopic image processing apparatus according to claim 9, wherein the information about the insertion status of the endoscope includes at least one of insertion length of the endoscope, elapsed time after insertion of the endoscope, and insertion shape of the endoscope.
